# EUROPEAN PATENT APPLICATION

(11) **EP 4 832 007 A1**
(43) Date of publication of application: **28.10.2026**
(21) Application number: 25172659.2
(22) Date of filing: 25.04.2025
(51) Int. Cl.: G16H 50/20, G16H 50/30, G16H 20/10, G16H 30/40, G16H 70/60, A61B 5/00, G06T 7/00, A61B 6/00

(54) **A METHOD FOR IDENTIFYING A CHRONIC INFLAMMATORY DISEASE**

(71) Applicant: HepaVue Dx, 1410 Waterloo (BE)
(72) Inventor: EL ALAM, Gaby, 1004 Lausanne (CH); NACHIT, Maxime, 7973 Stambruges (BE)
(74) Representative: Calysta NV

(57) **Abstract**

A method for generating a subset of imaging features predicting a chronic inflammatory disease comprising collecting imaging data of a target tissue or organ an of a control tissue or organ of at least one patient diagnosed with chronic inflammatory disease, said patient being pre-administrated with an imaging contrast agent composition comprising a molecule selectively binding to a cell adhesion molecule, wherein said molecule is conjugated to an imaging contrast agent, performing a segmentation of said imaging data, detecting a binding pattern of said imaging contrast agent, quantifying said binding pattern through a plurality of imaging features, processing said imaging features to represent signal variability, correlating said imaging features with said chronic inflammatory disease, generating said subset of imaging features by selecting features which exhibits discriminative power between said target and said control tissue or organ.

## Description

### Technical Field

The present invention relates to a method for generating a subset of imaging features predicting a chronic inflammatory disease.

The present invention also relates to a method for identifying a chronic inflammatory disease in a patient.

The present invention also relates to the treatment of a chronic inflammatory disease in a patient wherein the chronic inflammatory disease is identified by the method according to the present invention.

The present invention also relates to a therapeutic agent for use in the treatment of a chronic inflammatory disease in a patient wherein said chronic inflammatory disease is identified by the method for identifying a chronic inflammatory disease in a patient according to the present invention.

### State of the art

Chronic inflammatory diseases (CIDs) are a group of long-term conditions characterized by persistent inflammation that can damage tissues and organs over time. Unlike acute inflammation, which is a short-term immune response to infection or injury, chronic inflammation persists for months or years, often leading to progressive tissue damage and dysfunction. These diseases can affect various body systems, including the joints, skin, digestive tract, lungs, nervous system, and cardiovascular system.

CIDs can result from a variety of causes, including genetic predisposition, autoimmune reactions, persistent infections, environmental factors, and lifestyle influences such as diet, stress, and smoking. Some of the most common chronic inflammatory diseases include rheumatoid arthritis, psoriatic arthritis, ankylosing spondylitis, lupus, vasculitis (e.g. Takayasu arteritis, giant cell arteritis, ANCA-associated vasculitis, Beh 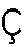 et's disease), atherosclerosis, polymyositis, dermatomyositis, systemic sclerosis, and Sjögren's syndrome in the joints, muscles, skin, and connective tissues; Crohn's disease, ulcerative colitis, eosinophilic esophagitis, celiac disease, and microscopic colitis in the gastrointestinal tract; asthma, chronic obstructive pulmonary disease (COPD), hypersensitivity pneumonitis, sarcoidosis, idiopathic pulmonary fibrosis (IPF), progressive pulmonary fibrosis (PPF), and cystic fibrosis in the lungs; chronic glomerulonephritis, lupus nephritis, and IgA nephropathy in the kidneys; chronic liver disease (viral, autoimmune, metabolic, alcoholic), primary biliary cholangitis, primary sclerosing cholangitis, and hepatic fibrosis; chronic pancreatitis (autoimmune, metabolic) in the pancreas; myocarditis, endocarditis, rheumatic heart disease, pericarditis, and cardiac fibrosis in the heart; uveitis, scleritis, and keratitis in the eyes; thyroiditis (Hashimoto's, Graves' disease) in the thyroid; interstitial cystitis in the bladder; chronic prostatitis in the prostate; endometriosis and chronic pelvic inflammatory disease (PID) in the reproductive organs; and chronic neuroinflammation in Alzheimer's, Parkinson's, multiple sclerosis, chronic traumatic encephalopathy (CTE) in the central nervous system (CNS), and chronic lung diseases.

The impact of chronic inflammation extends beyond localized symptoms, as it has been linked to an increased risk of conditions such as heart disease, diabetes, and neurodegenerative disorders. Diagnosis are often laborious and often requires a combination of clinical evaluation, laboratory tests, imaging studies, and biomarker analysis.

In particular, chronic liver disease (CLD), notably those caused by metabolic dysfunction and/or alcohol use disorder, follows a progressive spectrum characterized by liver fat accumulation and subsequent pathological changes. Both metabolic dysfunction-associated fatty liver disease (MAFLD, formerly NAFLD) and alcohol-associated liver disease (ALD) share the common feature of hepatic steatosis (fatty liver), which represents the initial stage of disease progression.

In both conditions, persistent metabolic or alcohol-induced stress can trigger hepatocyte injury and inflammation, leading to steatohepatitis-a state of fatty liver with inflammatory cell infiltration and hepatocellular damage. This inflammatory phase, known as metabolic dysfunction-associated steatohepatitis (MASH) in MASLD or alcoholic steatohepatitis (ASH) in ALD, can further stimulate fibrogenic pathways, resulting in the excessive deposition of extracellular matrix components. Over time, progressive fibrosis may lead to cirrhosis, characterized by severe architectural distortion and functional impairment of the liver. In advanced cases, this pathological remodeling significantly increases the risk of hepatocellular carcinoma (HCC).

High calories intake and sedentary lifestyle leading to CLD represent a major public health challenge due to their rising prevalence, driven by sedentary lifestyles, calorie-rich diets and/or alcohol consumption. Understanding the underlying mechanisms and developing targeted therapeutic strategies are critical priorities to prevent severe complications and improve patients' quality of life.

Steatohepatitis is an aggressive form of fatty liver disease, which is notably included in MAFLD as well as in the alcoholic liver disease (ALD) spectrum. Steatohepatitis has the inherent propensity to progress towards liver fibrosis which can produce progressive liver scarring and lead to cirrhosis or towards hepatocellular carcinoma (liver cancer). Liver cancer is the most common type of primary liver cancer in adults and it is the second leading cause of cancer-related death worldwide. Hepatocellular carcinoma (HCC) accounts for 70-85% of all liver cancers. MAFLD, the most prevalent chronic liver disease in the world, is an established risk factor for HCC development. The prevalence of MAFLD-associated HCC is alarmingly increasing. A study conducted in Newcastle (United Kingdom) reported an over ten-fold increase of MAFLD-associated HCC between 2000 and 2010. By 2030, HCC incidence is projected to rise by 137% in the United States with approximately 20% of these new cases related to MAFLD. Hence, tools to evaluate the risk for HCC development in MAFLD patients are urgently needed to optimize surveillance in this vast at-risk population.

MASH and ASH are generally diagnosed through the histological examination by an expert liver pathologist of a liver sample obtained by a biopsy. While liver biopsy is still considered the gold standard to assess the presence and/or the severity of a liver condition in a subject, it does have limitations, notably due to a poor inter- or intra-observer reproducibility and a possible sample bias linked to the small size of the sample. Furthermore, liver biopsy is an invasive medical procedure and as such remains associated with a risk of complication and a significant cost.

Consequently, there remains a need for a non-invasive method to specifically and accurately assess the presence of chronic inflammatory disease and chronic liver disease in particular, such as steatohepatitis and MASH, and/or a complication thereof in a patient. In particular, such a non-invasive method would represent a convenient and practical manner to assess the presence of chronic liver disease, and/or a complication thereof in a patient, to monitor patients suffering from chronic liver disease such as MAFLD, ALD, MASH or ASH, and to monitor treatment response in a patient treated for chronic liver disease.

It was shown that VCAM-1 is largely upregulated and is a key pathophysiological player in MASH. In that framework, a study indicated that VCAM-1 may be a suitable non-invasive mean to detect liver inflammation ahead of fibrosis development (Nachit & al., Molecular imaging of liver inflammation using an anti-VCAM-1 nanobody, Nature communications (2023)14:1062); hence VCAM-1 quantification was proposed as a mean to detect liver inflammation.

While this approach provides valuable insights, the quantification methods employed were relatively simple, typically relying on mean standardized uptake values (SUV) or percentage of injected dose (%ID), conventional metrics well-known by those skilled in the art. However, in real-world clinical scenarios, where patient populations exhibit significant biological variability, these conventional approaches may lack precision and robustness. Surprisingly, it has been found that more refined and non-obvious methods of signal quantification-leveraging advanced analytical models and image processing techniques-can yield superior performance. These methods not only enhance the accuracy and reproducibility of inflammatory signal quantification but also improve sensitivity in detecting subtle pathological changes that might otherwise be overlooked.

### Objectives of the invention

The present invention aims to overcome the drawbacks of the state of the art, in particular those described above.

In particular, the present invention proposes a method for generating a subset of features predicting a chronic inflammatory disease which is reliable, precise and non-invasive.

The present invention also proposes a method for identifying a chronic inflammatory disease in a patient that is non-invasive, without the need to resort to a biopsy, for example a liver biopsy, said method for identifying a chronic inflammatory disease implements the subset of features generated from the method for generating a subset of features predicting a chronic inflammatory disease according to the present invention.

The present invention also proposes a method which is reliable with a high sensitivity and specificity for detection of chronic inflammatory disease, while also being precise and able to detect different stages of development of chronic inflammatory disease.

The present invention also provides a method allowing for a rapid identification of chronic inflammatory disease in a patient rendering it easily applicable in a daily medical practices.

### Summary of the invention

To achieve the aforementioned objectives, the present invention provides a method for generating a subset of (imaging) features predicting a chronic inflammatory disease, the method comprising:
(i) collecting imaging data of a target, inflammatory, tissue or organ and of a control tissue or organ of at least one patient being diagnosed with said chronic inflammatory disease, said patient being pre-administrated with an imaging contrast agent composition comprising a molecule selectively binding to a cell adhesion molecule, wherein said molecule is conjugated to an imaging contrast agent, said imaging data comprising at least an image obtained from said imaging contrast agent (imaging probe signal) and a (native) image without said imaging contrast agent so as to spatially localize the said target tissue or organ and the said control tissue or organ,
(ii) correcting said imaging data for imaging probe signal attenuation,
(iii) performing a segmentation of said imaging data in order to select a region of interest within said target tissue or organ and within said control tissue or organ,
(iv) detecting a binding pattern of said imaging contrast agent within said region of interest of said target tissue or organ and of said control tissue or organ,
(v) quantifying said binding pattern through a plurality of imaging features to form an imaging dataset,
(vi) processing said imaging dataset to form a processed imaging dataset, said processed imaging dataset representing signal variability of said plurality of imaging features within said region of interest of said target tissue or organ and of said control tissue or organ,
(vii)correlating said processed imaging dataset of said target tissue or organ being diagnosed as a chronic inflammatory disease with said control tissue or organ being not diagnosed with said chronic inflammatory disease,
(viii) generating a subset of features by selecting at least one imaging feature and maximum 10, preferably maximum 8, 6, 5, 4, 3, 2, 1 imaging feature(s) from said processed imaging dataset which exhibits a discriminative power between said target tissue or organ being diagnosed as a chronic inflammatory disease and said control tissue or organ being not diagnosed with said chronic inflammatory disease, wherein said discriminative power is determined through an AUC (Area Under The Curve) ROC curve, having an AUC value of at least 0.8.

Indeed, the method for generating a subset of (imaging) features predicting a chronic inflammatory disease according to the present invention is centered on the detection of a binding pattern of the imaging contrast agent within a region of interest of a target, inflammatory, tissue or organ and of a control tissue or organ of at least one patient being diagnosed with said chronic inflammatory disease, wherein the imaging contrast agent comprises (or consists essentially of) a molecule selectively binding to a cell adhesion molecule such as for example vascular cell adhesion molecule-1 (VCAM-1), wherein said molecule is conjugated to a imaging contrast agent, and on a quantification of said binding pattern through a plurality of imaging features, and on a selection of a subset of imaging feature exhibiting correlation with said chronic inflammatory disease, wherein the subset of (imaging) features comprises at least one imaging feature and maximum 10 imaging features. The unique combination of the method according to the present invention allows determining a specific, minimal, subset of imaging features that can be used as a parametric, *in silico,* signature of said chronic inflammatory disease. The method according to the present invention allows generating a non-invasive, rapid, *in silico,* signature that can be used for identifying a chronic inflammatory disease.

Other embodiments of the method for generating a subset of (imaging) features predicting a chronic inflammatory disease are indicated in the appended claims.

The present invention also relates to a method for identifying a chronic inflammatory disease in a patient, the method comprising the following steps:
(i) collecting imaging data of a target tissue or organ of said patient, said patient being pre-administrated with an imaging contrast agent composition comprising a molecule selectively binding to cell adhesion molecule, preferably a vascular cell adhesion molecule-1 (VCAM-1), wherein said molecule is conjugated to an imaging contrast agent said imaging data comprising at least an image obtained with said imaging contrast agent (imaging probe signal) and a (native) image without said imaging contrast agent so as to spatially localize the said tissue or organ,
(ii) correcting said imaging data for imaging probe signal attenuation,
(iii) performing a segmentation of said imaging data in order to select a region of interest within said tissue,
(iv) detecting a binding pattern of said imaging contrast agent within said region of interest,
(v) quantifying said binding pattern through at least said subset of imaging features obtained from said method for generating a subset of (imaging) features according to the present invention,
(vi) identifying and providing a risk for said patient of having or developing said chronic inflammatory disease based on said quantification of said binding pattern

Indeed, the method for identifying a chronic inflammatory disease in a patient is centered on the collection of imaging data of a target tissue or argan of said patient being pre-administrated with an imaging contrast agent composition comprising a molecule selectively binding to a cell adhesion molecule such as for example vascular cell adhesion molecule-1 (VCAM-1), wherein said molecule is conjugated to an imaging contrast agent, on a detection of a binding pattern of said imaging contrast agent within a region of interest within said target tissue or organ, and on an organization of said binding pattern through a quantification of at least said subset of (imaging) features obtained from said method for generating a subset of (imaging) features according to the present invention. This method identifies and provides a risk for said patient of having or developing said chronic inflammatory disease based on said quantification of said binding pattern. The method according to the present invention, based on the analysis of the signal generated by a cell adhesion molecule such as VCAM-1 in the target tissue or organ of a patient, is reliable and precise allowing for an accurate identification with high confidence of a particular chronic inflammatory disease. The combined characteristics of the method according to the present invention permits having a rapid, precise, reliable and non-invasive method, making it a perfect diagnostic tool for a daily clinical routine.

Other embodiments of the method for identifying a chronic inflammatory disease in a patient according to the present invention are indicated in the appended claims.

The present invention also relates to a method for treating a chronic inflammatory disease, preferably a chronic liver disease, in a patient wherein said chronic inflammatory disease, preferably a chronic liver disease, is identified by the method for identifying a chronic inflammatory disease according to the present invention, wherein said method for treating a chronic inflammatory disease, preferably a chronic liver disease, comprises administrating a pharmaceutically effective amount of at least one therapeutic agent from the group of therapeutic agents consisting of Resmetirom, Semaglutide, Cenicriviroc, Saroglitazar, Emricasan, Belapectin, Simtuzumab, Ocaliva, Tropifexor, Lanifibranor, Pegbelfermin, VK2809, a combinaison of Tropifexor/CFZ533, Aldafermin, Aramchol, Namodenoson, Efruxifermin, Cotadutide, ALT-801, BI-456906, Mazdutide (LY3298176), BI-76741, GB1211, CB4211, PLN-74809, CC-90001, CRV431, CF102, BMS-986036, ASC41, THRβ agonist, MET642, TERN-101, EDP-305, BIO89-100, Pemafibrate, Seladelpar, DNL747, Rimonabant, Efruxifermin, Tirzepatide, Efinopegdutide, Survodutide to the patients diagnosed as at risk.

Other embodiments of the method for treating a chronic inflammatory disease in a patient according to the present invention are indicated in the appended claims.

The present invention also relates to a therapeutic agent selected from the group of therapeutic agents consisting of Resmetirom, Semaglutide, Cenicriviroc, Saroglitazar, Emricasan, Belapectin, Simtuzumab, Ocaliva, Tropifexor, Lanifibranor, Pegbelfermin, VK2809, a combinaison of Tropifexor/CFZ533, Aldafermin, Aramchol, Namodenoson, Efruxifermin, Cotadutide, ALT-801, BI-456906, Mazdutide (LY3298176), BI-76741, GB1211, CB4211, PLN-74809, CC-90001, CRV431, CF102, BMS-986036, ASC41, THRβ agonist, MET642, TERN-101, EDP-305, BIO89-100, Pemafibrate, Seladelpar, DNL747, Rimonabant, Efruxifermin, Tirzepatide, Efinopegdutide, survodutide for use in the treatment of a chronic inflammatory disease, preferably a chronic liver disease associated with metabolic dysfunction, in a patient wherein said chronic inflammatory disease is identified by the method for identifying a chronic inflammatory disease according to the present invention.

Other embodiments of the therapeutic agent for use in the treatment of a chronic inflammatory disease in a patient according to the present invention are indicated in the appended claims.

The present invention also relates to a molecule selectively binding to a cell adhesion molecule wherein said molecule is conjugated to a radioactive isotope, for use in a treatment of a tumor in order to potentiate a subsequent cancer treatments, including but not limited to immunotherapy approaches such as checkpoint inhibitors, including PD-1 inhibitors such as pembrolizumab (Keytruda) and nivolumab (Opdivo), PD-L1 inhibitors such as atezolizumab (Tecentriq) and durvalumab (Imfinzi), and CTLA-4 inhibitors such as ipilimumab (Yervoy). Other types of immunotherapy include cytokine therapy, such as interleukin-2 (IL-2) therapy with aldesleukin (Proleukin), interferon therapy such as interferon-alpha (Intron A), and therapies utilizing interleukin-15 (IL-15) or IL-12. Additionally, adoptive cell transfer (ACT) therapies may be used, including CAR-T cell therapy such as tisagenlecleucel (Kymriah) and axicabtagene ciloleucel (Yescarta), tumor-infiltrating lymphocyte (TIL) therapy, and TCR-engineered T cell therapy. The molecule may also be used with cancer vaccines, including peptide-based vaccines, DNA or RNA vaccines, dendritic cell vaccines such as sipuleucel-T (Provenge), and oncolytic virus vaccines such as talimogene laherparepvec (T-VEC). Monoclonal antibody therapy may also be employed, including bispecific T cell engagers (BiTEs) such as blinatumomab (Blincyto), antibody-drug conjugates (ADCs) such as brentuximab vedotin (Adcetris), and naked monoclonal antibodies such as rituximab (Rituxan). In addition, immune modulators may be utilized, including thalidomide and its derivatives such as lenalidomide (Revlimid) and pomalidomide (Pomalyst), as well as checkpoint agonists such as OX40 agonists and CD40 agonists, STING (stimulator of interferon genes) agonists, and toll-like receptor (TLR) agonists. Corticosteroids and derivatives may be used as adjunctive therapy to modulate immune responses or manage immune-related adverse events. The molecule may also be combined with other therapeutic approaches, such as combining immunotherapy with chemotherapy or targeted therapy, including BRAF or MEK inhibitors, or employing dual immune checkpoint blockade, such as combining nivolumab with ipilimumab.

### Detailed description of the invention

Other features and advantages of the present invention will be derived from the following non-limiting description, and with reference to the following examples.

According to the present invention, preferably, the term "quantifying a binding pattern" is understood to mean a quantification of a plurality of imaging features. Alternatively, or in addition to the quantification of a plurality of imaging feature, the term "quantifying a binding pattern" comprises a calculation of the standardized uptake value (SUV), also known as the dose uptake ratio (DUR) and is a mathematically derived ratio of tissue radioactivity concentration at a point in time C(T) at a specific region of interest (ROI) and the injected dose of radioactivity per kilogram of the patient's body weight: SUV = C(T)/[injection dose (MBq)/patient's weight (kg)].

According to the present invention, preferably, the term "significant discriminative power associated to said chronic inflammatory disease" is understood to mean an ability of a statistical test to differentiate between an inflammatory state (whether chronic or not, preferably chronic inflammatory state) and a non-inflammatory, normal, state. Preferably, the significant discriminative power is determined through an AUC (Area Under The Curve) of a ROC (Receiver Operating Characteristic) curve, having an AUC value of at least 0.8, more preferably at least 0.81, 0.82, 0.83, 0.84, 0.85, 0.86, 0.87, 0.88, 0.89, 0.90, or even 0.91, 0.92, 0.93, 0.94, 0.95, or even more 0.96, 0.97, 0.98, or 0.99

According to the present invention, preferably, the term "chronic inflammatory disease" means an inflammatory disease wherein at least one marker of the disease, such as for example an overexpression of an inflammatory marker, is detectable for at least 48h.

Preferably, the cell adhesion molecule is chosen in a group consisting of vascular cell adhesion molecule-1 (VCAM-1; CD106), Integrin a4 (VLA-4), Intercellular Adhesion Molecule-1 (ICAM-1 or CD54), Integrin a5, Intercellular Adhesion Molecule-2 (ICAM-2 or CD102), Integrin a6, PSGL-1, CD44, NCAM (CD56), ALCAM (CD166), E-selectin (CD62E), PECAM (CD31), P-selectin (CD62P), EpCAM (CD326), ICAM-3 (CD50), or L-selectin (CD62L). More preferably, the cell adhesion molecule is a vascular cell adhesion molecule-1 (VCAM-1).

According to the present invention, preferably, the term "correcting said imaging data for imaging probe signal attenuation" means a correction of signal loss (attenuation) as a consequence of tissue density. Indeed, denser tissues (like bone) attenuate more the signal of the contrast agent (i.e. the radionuclide decay) than soft tissues. Preferably, in Positron Emission Tomography - Computed Tomography (PET-CT) or Single Photon Emission Computed Tomography-Computed Tomography (SPECT-CT), the Computed Tomography image (CT image) provides density information (Hounsfield units) that directly correlates with the attenuation coefficients of tissues, allowing for accurate correction of signal loss (attenuation). Preferably, in Positron Emission Tomography - Magnetic Resonance Imaging PET-MRI) or SPECT-MRI, the MRI image does not provide direct density information but gives high-resolution spatial localization of tissues. Preferably, in presence of PET-MRI or SPECT-MRI, a correction for attenuation uses advanced methods like Al algorithms or tissue classification techniques to infer density based on the tissue type (e.g., bone, soft tissue) from the MRI data.

Preferably, the area under the curve is equal or superior to 0.8, preferably equal or superior to 0.85, 0.9, 0.91, 0.92, 0.93, 0.94, or even 0.95.

The method for generating a subset of (imaging) features predicting a chronic inflammatory disease comprises a step of collecting imaging data of a target tissue or an organ of a group of patients comprising at least one patient being diagnosed with said chronic inflammatory disease and at least one patient being not diagnosed with said chronic inflammatory disease, each of said patients being pre-administrated with an imaging contrast agent composition comprising a molecule selectively binding to a cell adhesion molecule, such as vascular cell adhesion molecule-1 (VCAM-1), wherein said molecule is conjugated to an imaging contrast agent, said imaging data comprising at least an image obtained with said imaging agent (imaging probe signal) and a (native) image without said imaging agent so as to spatially localize the said tissue or organ.

Preferably, said imaging contrast agent is chosen in the group consisting of radioactive isotope, an iron oxide nanoparticle, an encapsulated gas microbubble. More preferably, said imaging agent is a radioactive isotope.

Preferably, said tissue or organ is the liver, the intestine, the pancreas, joint tissue, muscle tissue, the kidneys, the brain, the eyes, skin tissue, the lungs, the heart, the blood vessels, the bone, the bone marrow, reproductive tissue, and/or the thyroid, more preferably said tissue or organ is the liver.

Preferably, for each patient of the group of patients, the imaging data of a target tissue or organ comprises at least the image obtained with the imaging contrast agent (imaging probe signal) and a (native) image of the target tissue or organ of the patient without the imaging contrast agent, preferably a 3-dimensional (3D) image. Preferably, the image obtained with the imaging contrast agent (imaging probe signal) and the (native) image of the target tissue or organ of the patient are merged.

Preferably, the imaging data of said target tissue or organ and of said control tissue or organ is collected on the same patient being diagnosed with said chronic inflammatory disease, wherein said target tissue or organ comprises at least an inflammatory zone of said target tissue or organ, and wherein said control tissue or organ comprises at least a non-inflammatory zone of said control tissue or organ, wherein said target tissue or organ and said control tissue or organ are the same tissue or organ or are different tissue or organ. Alternatively, the imaging data of said control tissue or organ is collected on at least one patient being not diagnosed with said chronic inflammatory disease. Preferably, said control tissue or organ collected on at least one patient being not diagnosed with said chronic inflammatory disease is the same type of tissue or organ than said target tissue or organ collected on the patient being diagnosed with said chronic inflammatory disease.

According to the present invention, preferably, said control tissue or organ from a patient being diagnosed with said chronic inflammatory disease or not is a control, healthy, tissue or organ.

Preferably or alternatively, the imaging data of said target tissue or organ and of said control tissue or organ is collected on a group of patients. Preferably, the group of patients comprises at least 2, 3, 4, 5, 10, 20, 30, 40, 50, 100, 200, or even at least 250 patients being diagnosed with said chronic inflammatory disease. Preferably, the group of patients comprises at least 2, 3, 4, 5, 10, 20, 30, 40, 50, 100, 200, or even at least 250 patients being diagnosed with no chronic inflammatory disease.

Preferably, the molecule is
- a small molecule having a molecular weight equal or inferior to 10000 Da, more preferably equal or inferior to 5000 Da, even more preferably equal or inferior to 2000 Da,
- an antibody, a monoclonal antibody, or
- a peptide, or
- a small scaffold protein, an antibody mimetic such as but not limited to single-domain antibodies (sdAbs), VHH (Variable Heavy-chain domain of Heavy-chain-only antibodies) derived from camelids, VNAR (Variable New Antigen Receptor) from sharks, ScFv (Single-Chain Variable Fragment) composed of fused VH and VL domains from conventional antibodies, Fab (Fragment antigen-binding) containing one constant and one variable domain from both heavy and light chains, F(ab')2 which is a dimer of Fab fragments linked by a disulfide bond, diabodies which are dimeric ScFv molecules with two antigen-binding sites, triabodies and tetrabodies which are multimeric ScFv formats, DARPins (Designed Ankyrin Repeat Proteins) engineered from ankyrin repeat proteins, affibodies which are small proteins derived from the Z-domain of Staphylococcal protein A, adnectins based on the fibronectin type III domain, anticalins which are engineered from lipocalins to bind specific targets, monobodies derived from the fibronectin type III domain, centyrins based on the human fibronectin type III domain scaffold, avimers composed of peptide domains derived from human extracellular receptor domains, knottins which are small, stable peptides with a cystine knot motif, repebodies based on leucine-rich repeat (LRR) proteins, scaffold-based peptides which are peptides derived from small, stable protein domains, and repebody which are engineered from repeat proteins for high specificity binding.

According to the present invention, preferably, the term "small molecule" is understood to mean an organic compound, usually hydrophobic, having a molecular weight preferably inferior to 10000 Da, more preferably inferior to 5000 Da, even more preferably equal or inferior to 2000 Da, wherein the small molecule is able to diffuse across the cell membrane and can target intracellular molecules.

Preferably, the radioactive isotope is selected from a group of radioactive isotope consisting of Gallium-68 (⁶⁸Ga), Fluorine-18 (¹⁸F), Carbon-11 (¹¹C), Nitrogen-13 (¹³N), Oxygen-15 (¹⁵O), Rubidium-82 (⁸²Rb), Zirconium-89 (⁸⁹Zr), Scandium-44 (⁴⁴Sc), Yttrium-86 (⁸⁶Y), Technetium-99m (^{99m}Tc), Iodine-123 (¹²¹I), Indium-111 (¹¹¹In), Gallium-67 (⁶⁷Ga), Thallium-201 (²⁰¹Tl), Xenon-133 (¹³³Xe), Iodine-131 (¹³¹I), Copper-64 (⁶⁴Cu).

Preferably, the molecule is a nanobody selectively binding to VCAM-1 and wherein the nanobody is conjugated to the radioactive isotope. More preferably, the nanobody selectively binding to VCAM-1 is conjugated to Gallium-68 (⁶⁸Ga) or to Fluorine-18 (¹⁸F),

The method for generating a subset of imaging features predicting a chronic inflammatory disease further comprises a step of performing a segmentation of said imaging data in order to select a region of interest within said target tissue or organ.

Preferably, said segmentation of said imaging data in order to select a region of interest within said tissue is performed on said (native) image without said imaging contrast agent composition, and preferably wherein at least 50% of the volume of the organ or tissue is segmented, preferably at least 60%, more preferably at least 70%, still more preferably at least 80%, 90%, 95% or (almost) 100% of the organ or tissue is segmented.

Preferably, said chronic inflammatory disease is rheumatoid arthritis, psoriatic arthritis, ankylosing spondylitis, lupus, vasculitis (e.g. Takayasu arteritis, giant cell arteritis, ANCA-associated vasculitis, Beh 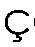 et's disease), atherosclerosis, polymyositis, dermatomyositis, systemic sclerosis, and Sjögren's syndrome in the joints, muscles, skin, and connective tissues; Crohn's disease, ulcerative colitis, eosinophilic esophagitis, celiac disease, and microscopic colitis in the gastrointestinal tract; asthma, chronic obstructive pulmonary disease (COPD), hypersensitivity pneumonitis, sarcoidosis, idiopathic pulmonary fibrosis (IPF), progressive pulmonary fibrosis (PPF), and cystic fibrosis in the lungs; chronic glomerulonephritis, lupus nephritis, and IgA nephropathy in the kidneys; chronic liver disease (viral, autoimmune, metabolic, alcoholic), primary biliary cholangitis, primary sclerosing cholangitis, and hepatic fibrosis; pancreatitis, for example chronic pancreatitis (autoimmune, metabolic) in the pancreas; myocarditis, endocarditis, rheumatic heart disease, pericarditis, and cardiac fibrosis in the heart; uveitis, scleritis, and keratitis in the eyes; thyroiditis (Hashimoto's, Graves' disease) in the thyroid; interstitial cystitis in the bladder; chronic prostatitis in the prostate; endometriosis and chronic pelvic inflammatory disease (PID) in the reproductive organs; and chronic neuroinflammation in Alzheimer's, Parkinson's, multiple sclerosis, and chronic traumatic encephalopathy (CTE) in the central nervous system (CNS), preferably a chronic liver disease associated with metabolic dysfunction.

Preferably, the segmentation is performed through an automatic image segmentation process. Preferably, the segmentation is a manual segmentation of the liver tissue and/or a software neural-network based segmentation of the inflammatory tissue or organ.

Preferably, the segmentation comprises a preliminary step of collecting at least one 3D image of the target inflammatory tissue or organ, in order to determine its boundaries and to extract one or more image-derived textural features (e.g., radiomic features or data) from the 3D image.

Preferably, the region of interest that is selected represents a large proportion of the target tissue or organ within the imaging data, for example the surface (or the volume) of the region of interest represents more than 20%, 30%, 40%, 50%, 60%, 70%, 80%, or even 90% of the surface (or the volume) of the target tissue or organ within the imaging data (surface, or volume, of the region of interest / surface, or volume, of the target tissue or organ within the imaging data).

The method for generating a subset of (imaging) features predicting a chronic inflammatory disease further comprises a step of detecting a binding pattern of said imaging contrast agent within said region of interest.

Preferably, the detecting step of the binding pattern of said imaging contrast agent to said liver tissue is performed in relying on the 3D liver images (see above), such as 3D images obtained by a positron emission tomography (PET). Indeed, a positron emission tomography (PET) imaging technique gives a very precise imaging data of high quality.

The method for generating a subset of (imaging) features predicting a chronic inflammatory disease further comprises a step of quantifying said binding pattern through a plurality of imaging features to form an imaging dataset. Preferably, quantifying said binding pattern through a plurality of imaging features to form an imaging dataset comprising performing a 3D quantification pattern of the plurality of imaging features, preferably a plurality of radiomic features.

Preferably, the plurality of imaging features comprises one or several radiomic features.

Preferably, the radiomic feature is chosen from a group of radiomic features consisting of first-order statistics features, shape features, texture features, wavelet features, filter-based features and/or fractal features.

Preferably, the first-order statistics features are chosen from a group of radiomic features consisting of mean, median, standard deviation, variance, skewness, kurtosis, energy, entropy, minimum, maximum, range, mode, interquartile range (IQR), mean absolute deviation (MAD), robust mean absolute deviation (rMAD), coefficient of variation (COV), root mean squared (RMS), and total energy, where if the mean or medium features are selected, this is necessarily with at least an additional feature from the above list.

Preferably, the shape radiomic features are chosen from a group of radiomic features consisting of volume, surface area, surface-to-volume ratio, compactness, sphericity, elongation, flatness, major axis length, minor axis length, least axis length, maximum 3D diameter, equivalent diameter, bounding box dimensions, mesh volume, surface area density, and solidity.

Preferably, said subset of features further comprises at least one clinical parameter chosen from a group consisting of physiological parameters, anthropometric parameters, biological parameters, cardiovascular parameters, and/or respiratory parameters.

Preferably, the texture radiomic features, derived from matrices that capture spatial relationships of voxel intensities, are chosen from a group of radiomic features consisting of Gray Level Co-occurrence Matrix (GLCM) such as for example contrast, correlation, angular second moment (ASM), homogeneity, entropy, dissimilarity, autocorrelation, cluster prominence, and cluster shade; Gray Level Run Length Matrix (GLRLM) such as for example long run emphasis (LRE), short run emphasis (SRE), run percentage (RP), gray level non-uniformity (GLNU), run length non-uniformity (RLNU), low gray-level run emphasis (LGRE), and high gray-level run emphasis (HGRE); Gray Level Size Zone Matrix (GLSZM) such as for example small area emphasis (SAE), large area emphasis (LAE), gray level non-uniformity (GLNU), size zone non-uniformity (SZNU), zone percentage (ZP), low gray-level zone emphasis (LGZE), and high gray-level zone emphasis (HGZE); Neighbouring Gray Tone Difference Matrix (NGTDM) such as for example coarseness, contrast, busyness, complexity, and strength.

Preferably, the wavelet features, derived from decomposing an image into frequency sub-bands using wavelet transforms, are chosen from a group of radiomic features consisting of mean, median, standard deviation, variance, energy, entropy, skewness, kurtosis, mean absolute deviation (MAD), root mean squared (RMS), interquartile range (IQR), and total energy.

Preferably, the filter-based features are chosen from a group of radiomic features consisting of Laplacian of Gaussian (LoG), Gabor filters, Gaussian filters, high-pass filters, and low-pass filters.

Preferably, the fractal features are chosen from a group of radiomic features consisting of Fractal Dimension (FD) and Box-Counting Fractal Dimension.

The method for generating a subset of imaging features predicting a chronic inflammatory disease further comprises a step of processing said imaging dataset to form a processed imaging dataset, said processed imaging dataset representing signal variability of said plurality of imaging features within said region of interest.

Preferably, said processing of said imaging dataset to form a processed imaging dataset comprises an implementation of a statistical method to reduce the dimensions of the imaging dataset. More preferably, processing of said imaging dataset comprises a dimension reduction performed using a principal component analysis (PCA) by keeping preferably the first P principal components, wherein P is comprised between 8 and 15.

Preferably, said processed imaging dataset is formed by selecting the principal components based on either (i) a minimum threshold of total variance explained (e.g., 40%, 50%, or 80%), (ii) their predictive power in diagnosing said chronic inflammatory disease, or (iii) both, thereby ensuring that components with lower variance yet high predictive relevance are also retained.

The method for generating a subset of imaging features predicting a chronic inflammatory disease further comprises a step of correlating said processed imaging dataset with said chronic inflammatory disease with said patient being diagnosed with said chronic inflammatory disease and with said patient being not diagnosed with said chronic inflammatory disease.

The method for generating a subset of imaging features predicting a chronic inflammatory disease further comprises a step of generating a subset of features by selecting at least one imaging feature and maximum 6, preferably maximum 5, 4, 3, 2, 1 imaging features from said processed imaging dataset which exhibits significant discriminative power associated to said chronic inflammatory disease.

Preferably, the step of correlating said processed imaging dataset with said chronic inflammatory disease and/or the step of generating said subset of imaging features by selecting at least one imaging feature and maximum 10, preferably maximum 8, 6, 5, 4, 3, 2, 1 imaging features from said processed imaging dataset is performed through predictive algorithms such as for example a logistic regression, a decision trees and random forest algorithm, a neural networks, etc...

The present invention also relates to a method for identifying a chronic inflammatory disease in a patient, the method comprising the following steps:
(i) collecting imaging data of a target tissue or organ and a control tissue or organ of at least one patient being diagnosed with said chronic inflammatory disease, said patient being pre-administrated with an imaging contrast agent composition comprising a molecule selectively binding to a cell adhesion molecule, such as vascular cell adhesion molecule-1 (VCAM-1), wherein said molecule is conjugated to an imaging contrast agent, said imaging data comprising at least an image obtained from said imaging contrast agent (imaging probe signal) and a (native) image without said imaging contrast agent so as to spatially localize the said tissue or organ,
(ii) correcting said imaging data for imaging probe signal attenuation,
(iii) performing a segmentation of said imaging data in order to select a region of interest within said tissue,
(iv) detecting a binding pattern of said imaging contrast agent within said region of interest,
(v) quantifying said binding pattern through at least said subset of imaging features obtained from said method for generating a subset of imaging features according to the present invention,
(vi) identifying and providing a risk for said patient of having or developing said chronic inflammatory disease based on said quantification of said binding pattern.

Preferably, said detecting step of the binding of said imaging contrast agent to said target tissue or organ, preferably a the liver, is performed by a positron emission tomography device (PET).

Preferably, the radioactive isotope is selected from a group of radioactive isotope consisting of Gallium-68 (⁶⁸Ga), Fluorine-18 (¹⁸F), Carbon-11 (¹¹C), Nitrogen-13 (¹³N), Oxygen-15 (¹⁵O), Rubidium-82 (⁸²Rb), Zirconium-89 (⁸⁹Zr), Scandium-44 (⁴⁴Sc), Yttrium-86 (⁸⁶Y), Technetium-99m (^{99ₘ}Tc), Iodine-123 (¹²¹I), Indium-111 (¹¹¹In), Gallium-67 (⁶⁷Ga), Thallium-201 (²⁰¹Tl), Xenon-133 (¹³³Xe), lodine-131 (¹³¹I), Copper-64 (⁶⁴Cu). Preferably, the molecule is a nanobody selectively binding to VCAM-1 and wherein the nanobody is conjugated to the radioactive isotope. More preferably, the nanobody selectively binding to VCAM-1 is conjugated to Gallium-68 (⁶⁸Ga) or to Fluorine-18 (¹⁸F).

Preferably, the method for identifying a chronic inflammatory disease in a patient according to the present invention comprises performing each step (i) to (v) of the method before a treatment and after said treatment, to assess changes in the development of said chronic inflammatory disease, for example in the inflammatory state of the disease.

Preferably, the chronic inflammatory disease is a chronic liver disease with a steatohepatitis component. Preferably, the chronic liver disease falls within a broader spectrum of steatosis-associated disorders, encompassing metabolic dysfunction and alcohol-related liver disease (MetALD), Metabolic-Associated Fatty Liver Disease (MAFLD), Alcohol-Associated Liver Disease (ALD). Even more preferably, the chronic liver disease is part of a group of conditions characterized by steatosis-driven inflammation and fibrotic progression, including Metabolic-Associated Steatohepatitis (MASH, formerly NASH) and Alcohol-Associated Steatohepatitis (ASH) and their progression to fibrosis, cirrhosis and hepatocellular carcinoma (HCC)

Preferably, at least one step of the method according to the present invention is a computer-assisted step. Preferably, collecting imaging data of the target tissue or organ of said patient is a computer-assisted step. Preferably, performing a segmentation of said imaging data is a computer-assisted step. Preferably, detecting a binding pattern of the imaging contrast agent within the region of interest is a computer-assisted step. Preferably, quantifying said binding pattern through a plurality of imaging features to form an imaging dataset is a computer-assisted step. Preferably, processing said imaging dataset to form a processed imaging dataset, said processed imaging dataset representing signal variability of said plurality of imaging features within said region of interest is a computer-assisted step. Preferably, correlating said processed imaging dataset with said chronic inflammatory disease with said patient being diagnosed with said chronic inflammatory disease and with said patient being not diagnosed with said chronic inflammatory disease is a computer-assisted step. Preferably, generating said subset of imaging features is a computer-assisted step. Preferably, identifying and providing a risk for said patient of having or developing said chronic inflammatory disease based on said quantification of said binding pattern is a computer-assisted step.

The present invention also relates to a method for treating a chronic inflammatory disease, preferably a chronic liver disease associated with metabolic dysfunction, in a patient wherein said chronic liver disease is identified by the method for identifying a chronic inflammatory disease associated with metabolic dysfunction in a patient according to the present invention, wherein said method for treating said chronic inflammatory disease comprises administrating a pharmaceutically effective amount of at least one therapeutic agent from the group of therapeutic agents consisting of Resmetirom, Semaglutide, Cenicriviroc, Saroglitazar, Emricasan, Belapectin, Simtuzumab, Ocaliva, Tropifexor, Lanifibranor, Pegbelfermin, VK2809, a combinaison of Tropifexor/CFZ533, Aldafermin, Aramchol, Namodenoson, Efruxifermin, Cotadutide, ALT-801, BI-456906, Mazdutide (LY3298176), BI-76741, GB121 1, CB421 1, PLN-74809, CC-90001, CRV431, CF102, BMS-986036, ASC41, THRβ agonist, MET642, TERN-101, EDP-305, BIO89-100, Pemafibrate, Seladelpar, DNL747, Rimonabant, Efruxifermin, Tirzepatide, Efinopegdutide, survodutide.

Preferably, the group of therapeutic agents consists of Resmetirom (CAS registry number : 920509-32-6), Semaglutide (CAS registry number : 910463-68-2), Cenicriviroc (CAS registry number : 497223-25-3, 497223-28-6), Saroglitazar (CAS registry number : 495399-09-2), Emricasan (CAS registry number : 254750-02-2), Belapectin (CAS registry number : 1980787-47-0, 1604015-18-0), Simtuzumab (CAS registry number : 1318075-13-6), Obeticholic acid (Ocaliva, CAS registry number : 459789-99-2), Tropifexor (CAS registry number : 1383816-29-2), Lanifibranor (CAS registry number : 927961-18-0), Pegbelfermin (CAS registry number : 2089039-22-3), VK2809 (CAS registry number : 852948-13-1), a combination of Tropifexor and CFZ533 (CAS registry numbers : 1383816-29-2 and 2031153-61-2), Aldafermin (CAS registry number : 1616639-03-2), Aramchol (CAS registry number : 246529-22-6), Namodenoson (CAS registry number : 163042-96-4), Efruxifermin (CAS registry number : 2375240-92-7), Cotadutide (CAS registry number : 1686108-82-6), ALT-801 (CAS registry number : 1463544-61-7), BI-456906 (CAS registry number : 2477737-80-5), Mazdutide (CAS registry number : 2259884-03-0), Tirzepatide (LY3298176) (CAS registry number : 2023788-19-2), BI-76741, Selvigaltin (GB1211) (UNII : 94EN2E6BLW), CB421 1, Bexotegrast (PLN-74809) (CAS registry number : 2376257-44-0), CC-90001 (CAS registry number : 1403859-14-2), Rencofilstat (CRV431) (CAS registry number : 1383420-08-3), CF102 (CAS registry number : 163042-96-4), Pegbelfermin (BMS-986036) (CAS registry number : 2089039-22-3), ASC41, THRβ agonists, Omesdafexor (MET642) (CAS registry number : 2244440-85-3), TERN-101 (CAS registry number : 1103500-20-4), EDP-305 (CAS registry number : 1933507-63-1), Pegozafermin (BIO89-100) (CAS registry number : 2489589-60-6), Pemafibrate (CAS registry number : 848259-27-8), Seladelpar (CAS registry number : 851528-79-5), DNL747, Rimonabant (CAS registry number : 168273-06-1).

Preferably, in method for treating a chronic inflammatory disease according to the present invention, the administrating step comprises an administration of a pharmaceutically effective amount of at least two therapeutic agents from said group of therapeutic agents, wherein said administration of a pharmaceutically effective amount of at least two therapeutic agents is performed sequentially or simultaneously.

Preferably, the method for treating a chronic inflammatory disease according to the present invention, further comprises a monitoring step of the outcomes of said patient by implementing, after said administrating step of a pharmaceutically effective amount of at least one therapeutic agent, said method for identifying a chronic inflammatory disease associated with metabolic dysfunction within said patient.

Preferably, said therapeutic agent is Resmetirom, preferably at a dosage comprised between 80 mg/day and 100 mg/day for at least 365 days.

Preferably, or alternatively, said therapeutic agent is Semaglutide or Lanifibranor, or a combination of Semaglutide and Lanifibranor, preferably wherein Semaglutide is administrated subcutaneous and is comprised at a dosage comprised between 1.5 mg and 3 mg, preferably around 2.4 mg, once a week, or at a dosage comprised between 0.2 mg and 0.6 mg, preferably around 0.4 mg, once a day.

Preferably, the therapeutic agent according to the present invention selected from the group of therapeutic agents consisting of Resmetirom, Semaglutide, Cenicriviroc, Saroglitazar, Emricasan, Belapectin, Simtuzumab, Ocaliva, Tropifexor, Lanifibranor, Pegbelfermin, VK2809, a combinaison of Tropifexor/CFZ533, Aldafermin, Aramchol, Namodenoson, Efruxifermin, Cotadutide, ALT-801, BI-456906, Mazdutide (LY3298176), BI-76741, GB1211, CB4211, PLN-74809, CC-90001, CRV431, CF102, BMS-986036, ASC41, THRβ agonist, MET642, TERN-101, EDP-305, BIO89-100, Pemafibrate, Seladelpar, DNL747, Rimonabant, Efruxifermin, Tirzepatide, Efinopegdutide, survodutide is for use in the treatment of steatohepatitis and/or NASH in a patient wherein said steatohepatitis and/or NASH is identified by the method according to the present invention.

### Examples.-

### Example 1.- Method according to the present invention for identifying a MASH disease in a patient

The method for identifying a chronic liver disease according to the present invention have been performed on a patient suspected of a MASH disease. The patient has been previously pre-administrated with an imaging contrast agent composition comprising a nanobody selectively binding to vascular cell adhesion molecule-1 (VCAM-1), wherein said nanobody is conjugated to ⁶⁸Ga. The imaging data of a liver tissue of said patient have been (1) collected and analyzed by (2) detecting the binding of said imaging contrast agent to said liver tissue through a positron emission tomography (PET) imaging technique, (3) quantifying the binding intensity, (4) comparing the binding intensity to a predetermined inflammatory threshold. The imaging confirms liver inflammation, and said MASH disease was (5) identified since said binding intensity is higher than said predetermined inflammatory threshold. All the steps (1) to (5) were computer-assisted steps.

### Example 2.- Treatment of a patient with a MASH disease wherein the MASH disease was diagnosed by the method for identifying a chronic liver disease according to the present invention.

The patient from Example 1 who was diagnosed with a MASH disease by implementing the method for identifying a chronic liver disease according to the present invention was treated with Resmetirom, an approved THR-β agonist. The patient has responded well to the treatment since liver fat and inflammation were reduced after the treatment.

### Example 3.- Method according to the present invention for identifying a MASH disease in a patient

The method for identifying a chronic liver disease according to the present invention described in the Example 1 was reproduced except that the patient has been previously pre-administrated with an imaging contrast agent composition comprising a first imaging contrast agent being a nanobody selectively binding to vascular cell adhesion molecule-1 (VCAM-1) wherein said nanobody is conjugated to ⁶⁸Ga. As in the Example 1, the imaging data of a liver tissue of said patient have been (1) collected and analyzed by (2) detecting the binding of the first and second imaging contrast agent to said liver tissue through a positron emission tomography (PET) imaging technique, (3) quantifying the binding intensities of the first and second imaging contrast agents (first binding intensity and second binding intensity respectively), (4) comparing the first binding intensity to a predetermined inflammatory threshold and the second binding intensity to a predetermined fibrosis threshold, and said MASH disease was (5) identified since said first binding intensity is higher than said predetermined inflammatory threshold while said second binding intensity is higher than the predetermined fibrosis threshold. The identification confirms inflammation and fibroblast activation associated with fibrosis stage F3. All the steps (1) to (5) were computer-assisted steps.

### Example 4.- Treatment of a patient with a MASH disease wherein the MASH disease was diagnosed by the method for identifying a chronic liver disease according to the present invention.

The patient from Example 3 that was diagnosed with a MASH disease by implementing the method for identifying a chronic liver disease according to the present invention was treated with a combination of Semaglutide and Lanifibranor to target inflammation, fat accumulation, and fibrosis simultaneously.

It should be understood that the present invention is not limited to the described embodiments and that variations can be applied without going outside of the scope of the claims.

## Claims

1. A method for generating a subset of imaging features predicting a chronic inflammatory disease, the method comprising:
(i) collecting imaging data of a target tissue or organ and of a control tissue or organ of at least one patient being diagnosed with said chronic inflammatory disease, said patient being pre-administrated with an imaging contrast agent composition comprising a molecule selectively binding to a cell adhesion molecule, wherein said molecule is conjugated to an imaging contrast agent, said imaging data comprising at least an image obtained from said imaging contrast agent (imaging probe signal) and a (native) image without said imaging contrast agent so as to spatially localize the said target tissue or organ and the said control tissue or organ,
(ii) correcting said imaging data for imaging probe signal attenuation,
(iii) performing a segmentation of said imaging data in order to select a region of interest within said target tissue or organ and within said control tissue or organ,
(iv) detecting a binding pattern of said imaging contrast agent within said region of interest of said target tissue or organ and of said control tissue or organ,
(v) quantifying said binding pattern through a plurality of imaging features to form an imaging dataset,
(vi) processing said imaging dataset to form a processed imaging dataset, said processed imaging dataset representing signal variability of said plurality of imaging features within said region of interest of said target tissue or organ and of said control tissue or organ,
(vii)correlating said processed imaging dataset of said target tissue or organ being diagnosed as a chronic inflammatory disease with said control tissue or organ being not diagnosed with said chronic inflammatory disease,
(viii) generating a subset of features by selecting at least one imaging feature and maximum 10, preferably maximum 8, 6, 5, 4, 3, 2, 1 imaging feature(s) from said processed imaging dataset which exhibits a discriminative power between said target tissue or organ being diagnosed as a chronic inflammatory disease and said control tissue or organ being not diagnosed with said chronic inflammatory disease, wherein said discriminative power is determined through an AUC (Area Under The Curve) ROC curve, having an AUC value of at least 0.8.

2. The method for generating a subset of imaging features predicting a chronic inflammatory disease according to claim 1, wherein said tissue or organ is the liver, the intestine, the pancreas, joint tissue, muscle tissue, the kidneys, the brain, the eyes, skin tissue, the lungs, the heart, the blood vessels, the bone, the bone marrow, reproductive tissue, and/or the thyroid, more preferably said tissue or organ is the liver.

3. The method for generating a subset of imaging features predicting a chronic inflammatory disease according to claim 1 or claim 2, wherein said segmentation of said imaging data in order to select a region of interest within said tissue is performed on said (native) image without said imaging contrast agent composition, and preferably wherein at least 50% of the volume of the organ or tissue is segmented, preferably at least 60%, more preferably at least 70%, still more preferably at least 80%, 90%, 95% or (almost) 100% of the organ or tissue is segmented.

4. The method for generating a subset of imaging features predicting an chronic inflammatory disease according to anyone of the preceding claims, wherein said chronic inflammatory disease is rheumatoid arthritis, psoriatic arthritis, ankylosing spondylitis, lupus, vasculitis (e.g. Takayasu arteritis, giant cell arteritis, ANCA-associated vasculitis, Beh 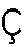 et's disease), atherosclerosis, polymyositis, dermatomyositis, systemic sclerosis, Sjögren's syndrome, muscles, skin, and connective tissues; Crohn's disease, ulcerative colitis, eosinophilic esophagitis, celiac disease, and microscopic colitis; asthma, chronic obstructive pulmonary disease (COPD), hypersensitivity pneumonitis, sarcoidosis, idiopathic pulmonary fibrosis (IPF), progressive pulmonary fibrosis (PPF), and cystic fibrosis; chronic glomerulonephritis, lupus nephritis, and IgA nephropathy; chronic liver disease (viral, autoimmune, metabolic, alcoholic), primary biliary cholangitis, primary sclerosing cholangitis, and hepatic fibrosis; pancreatitis (autoimmune, metabolic, and/or chronic); myocarditis, endocarditis, rheumatic heart disease, pericarditis, and cardiac fibrosis; uveitis, scleritis, and keratitis in the eyes; thyroiditis (Hashimoto's, Graves' disease); interstitial cystitis; chronic prostatitis; endometriosis and chronic pelvic inflammatory disease (PID); and chronic neuroinflammation in Alzheimer's, Parkinson's, multiple sclerosis, and chronic traumatic encephalopathy (CTE), preferably a chronic liver disease associated with metabolic dysfunction.

5. The method for generating a subset of imaging features predicting a chronic inflammatory disease according to any of the preceding claims, wherein said processing of said imaging dataset comprises a dimension reduction performed using a principal component analysis (PCA) by keeping preferably the first P principal components, wherein P is comprised between 8 and 15.

6. The method for generating a subset of imaging features predicting a chronic inflammatory disease according to any of the preceding claims, wherein said subset of features further comprises at least one clinical parameter chosen from a group consisting of physiological parameters, anthropometric parameters, biological parameters, cardiovascular parameters, and/or respiratory parameters.

7. The method for generating a subset of imaging features predicting an inflammatory disease according to any of the preceding claims, wherein said plurality of imaging features comprises one or several radiomic features chosen from a group of imaging features consisting of first-order statistics features, shape features, texture features, wavelet features, filter-based features and/or fractal features.

8. A method for identifying a chronic inflammatory disease in a patient, the method comprising the following steps:
(i) collecting imaging data of a target tissue or organ of said patient, said patient being pre-administrated with an imaging contrast agent composition comprising a molecule selectively binding to cell adhesion molecule, preferably a vascular cell adhesion molecule-1 (VCAM-1), wherein said molecule is conjugated to an imaging contrast agent said imaging data comprising at least an image obtained with said imaging contrast agent (imaging probe signal) and a (native) image without said imaging contrast agent so as to spatially localize the said tissue or organ,
(ii) correcting said imaging data for imaging probe signal attenuation,
(iii) performing a segmentation of said imaging data in order to select a region of interest within said tissue,
(iv) detecting a binding pattern of said imaging contrast agent within said region of interest,
(v) quantifying said binding pattern through at least said subset of imaging features obtained from said method for generating a subset of imaging features according to any of the preceding claims,
(vi) identifying and providing a risk for said patient of having or developing said chronic inflammatory disease based on said quantification of said binding pattern.

9. The method for generating a subset of imaging features predicting a chronic inflammatory disease according to any of the claims 1 to 7 or the method for identifying a chronic inflammatory disease in a patient according to claim 8, wherein said detecting step of the binding of said imaging contrast agent to said tissue or organ is performed by a positron emission tomography (PET).

10. The method for generating a subset of imaging features predicting a chronic inflammatory disease according to any of the claims 1 to 7 or the method for identifying a chronic inflammatory disease in a patient according to any of the claims 8 or claim 9, wherein the cell adhesion molecule is chosen in a group consisting of vascular cell adhesion molecule-1 (VCAM-1), Integrin a4 (VLA-4), Intercellular Adhesion Molecule-1 (ICAM-1 or CD54), Integrin a5, CD44, Intercellular Adhesion Molecule-2 (ICAM-2 or CD102), Integrin a6, PSGL-1, CD56, CD166, CD106, CD62E, CD31, CD62P, EpCAM (CD326), ICAM-3 (CD50), or L-selectin (CD62L), preferably the cell adhesion molecule is a vascular cell adhesion molecule-1 (VCAM-1).

11. The method for generating a subset of imaging features predicting a chronic inflammatory disease according to any of the claims 1 to 7 or the method for identifying a chronic inflammatory disease in a patient according to any of the claims 8 to 10, wherein said imaging contrast agent is chosen in the group consisting of radioactive isotope, an iron oxide nanoparticle, an encapsulated gas microbubble, preferably, said imaging agent is a radioactive isotope.

12. The method for generating a subset of imaging features predicting a chronic inflammatory disease according to any of the claims 1 to 7 or the method for identifying a chronic inflammatory disease in a patient according to any of the claims 8 to 11, wherein said imaging contrast agent is a radioactive isotope selected from the group consisting of Gallium-68 (⁶⁸Ga), Fluorine-18 (¹⁸F), Carbon-11 (¹¹C), Nitrogen-13 (¹³N), Oxygen-15 (¹⁵O), Rubidium-82 (⁸²Rb), Zirconium-89 (⁸⁹Zr), Scandium-44 (⁴⁴Sc), Yttrium-86 (⁸⁶Y), Technetium-99m (^{99ₘ}Tc), lodine-123 (¹²¹I), Indium-111 (¹¹¹In), Gallium-67 (⁶⁷Ga), Thallium-201 (²⁰¹Tl), Xenon-133 (¹³³Xe), lodine-131 (¹³¹I), Copper-64 (⁶⁴Cu).

13. The method for identifying a chronic inflammatory disease in a patient according to any of the claims 8 to 12, comprising performing each step (i) to (v) before a treatment and after said treatment, to as to assess changes the development of said chronic inflammatory disease.

14. The method for generating a subset of imaging features predicting a chronic inflammatory disease according to any of the claims 1 to 7 or the method for identifying a chronic inflammatory disease in a patient according to any of the claims 8 to 13, wherein at least one step, preferably each step, is a computer-assisted step.

15. A therapeutic agent selected from the group consisting of Resmetirom, Semaglutide, Cenicriviroc, Saroglitazar, Emricasan, Belapectin, Simtuzumab, Ocaliva, Tropifexor, Lanifibranor, Pegbelfermin, VK2809, a combinaison of Tropifexor/CFZ533, Aldafermin, Aramchol, Namodenoson, Efruxifermin, Cotadutide, ALT-801, BI-456906, Mazdutide (LY3298176), BI-76741, GB1211, CB4211, PLN-74809, CC-90001, CRV431, CF102, BMS-986036, ASC41, THRβ agonist, MET642, TERN-101, EDP-305, BIO89-100, Pemafibrate, Seladelpar, DNL747, Rimonabant, Efruxifermin, Tirzepatide, Efinopegdutide, survodutide for use in the treatment of a chronic inflammatory disease in a patient wherein said chronic inflammatory disease is identified by the method according to any of the claims 8 to 14.

16. A method for treating a chronic inflammatory disease associated with metabolic dysfunction in a patient wherein said chronic inflammatory disease is identified by the method according to any of claims 8 to 14, wherein said method for treating said chronic inflammatory disease comprises administrating a pharmaceutically effective amount of at least one therapeutic agent from the group consisting of Resmetirom, Semaglutide, Cenicriviroc, Saroglitazar, Emricasan, Belapectin, Simtuzumab, Ocaliva, Tropifexor, Lanifibranor, Pegbelfermin, VK2809, a combinaison of Tropifexor/CFZ533, Aldafermin, Aramchol, Namodenoson, Efruxifermin, Cotadutide, ALT-801, BI-456906, Mazdutide (LY3298176), BI-76741, GB1211, CB4211, PLN-74809, CC-90001, CRV431, CF102, BMS-986036, ASC41, THRβ agonist, MET642, TERN-101, EDP-305, BIO89-100, Pemafibrate, Seladelpar, DNL747, Rimonabant, Efruxifermin, Tirzepatide, Efinopegdutide, survodutide.

17. The method for treating a chronic inflammatory disease according to claim 16, wherein said administrating step comprises an administration of a pharmaceutically effective amount of at least two therapeutic agents from said group of therapeutic agents, wherein said administration of a pharmaceutically effective amount of at least two therapeutic agents is performed sequentially or simultaneously.

18. The method for treating a chronic inflammatory disease according to claim 16 or claim 17, wherein said therapeutic agent is Resmetirom, preferably at a dosage comprised between 80 mg/day and 100 mg/day for at least 365 days.

19. The method for treating a chronic inflammatory disease according to any of the claims 16 to 18, wherein said therapeutic agent is Semaglutide or Lanifibranor, or a combination of Semaglutide and Lanifibranor, preferably wherein Semaglutide is comprised at a dosage comprised between 2.4 mg/week and 3 mg/week for at least 52 weeks.
